# EUROPEAN PATENT APPLICATION

(11) **EP 3 851 852 A1**
(43) Date of publication of application: **21.07.2021**
(21) Application number: 20152470.9
(22) Date of filing: 17.01.2020
(51) Int. Cl.: G01N 33/50, G01N 33/68

(54) **METHOD FOR SEPARATION OF SPERM WITH UNDAMAGED INTACT HEADS FROM SPERM WITH DAMAGED HEADS AND SOMATIC CELLS**

(71) Applicant: Biotechnologicky Ustav AV CR, v.v.i., 25250 Vestec (CZ); PrimeCell Advanced Therapy, a.s., 11000 Praha 1 (CZ); Ceská zemedelská univerzita v Praze, 165 00 Praha 6 (CZ)
(72) Inventor: Komrskova, Katerina, 14900 Praha 4 (CZ); Postlerova, Pavla, 25264 Velke Prilepy (CZ); Frolikova, Michaela, 25101 Ricany (CZ); Zahradnicek, Michal, 11000 Praha 1 (CZ); Pospisilova, Veronika, 74101 Novy Jicin (CZ); Forostyak, Serhiy, 18000 Praha 8 (CZ); Simonik, Ondrej, 75366 Hustopece nad Becvou (CZ)
(74) Representative: Hartvichova, Katerina

(57) **Abstract**

The present invention provides a method of separation of sperm cells with undamaged intact heads from sperm cells with damaged heads and/or somatic cells and/or cell debris in a cell sample obtained from an animal, comprising the steps of:
- providing an anti-CD46 antibody bound to a carrier and/or to a tag;
- contacting the cell sample with the anti-CD46 antibody bound to the carrier and/or to the tag in order to bind CD46 presenting cells and/or cell debris from the cell sample to the anti-CD46 antibody;
- removing the CD46 presenting cells bound via the anti-CD46 antibody to the carrier and/or to the tag to obtain a cell sample free of CD46 presenting cells, wherein CD46 presenting cells include sperm cells with damaged heads, somatic cells and cell debris.

This technology is designed to separate sperm with undamaged intact heads to be further used in assisted reproduction, medicine (in particular breeding programs and veterinary medicine), storage purpose, and research.

## Description

### Field of Art

The present invention relates to a method of separation of sperm with undamaged intact heads from sperm with damaged heads, as well as from somatic cells and cell debris, in cell-containing samples obtained from an animal, such as ejaculate or semen sample. This method enables, for example, to use the separated sperm with intact heads in assisted reproduction, animal breeding programs and veterinary medicine to enhance the fertility success.

### Background Art

Artificial fertilization is a method widely used in the treatment of reproductive disorders by means of assisted reproduction as well as in agriculture, animal breeding programs and veterinary medicine. In all applications of artificial fertilization, the sperm quality is of crucial importance for achieving high fertilization success rates. Technologies for improvement of sperm quality are therefore being developed and they are of high priority. Some of the available methods for sperm quality improvement rely on health regimens and/or dietary improvements implemented by the males. Other methods focus on improving of sperm quality by enriching the sample in healthy sperm. A representative of such method is the method described in US 2017/0191029 in which a sperm sample is contacted with an insoluble carrier, which binds to an indicator of impaired sperm membrane integrity or impaired sperm motility. However only commercially available beads were tested in this document, some of which allowed achieving approximately 20% increase in motility, where for example, anti-rabbit IgG seemed to improve the ratio of healthy sperm.

CD46 (membrane cofactor protein) is membrane-associated glycoprotein that is present in human cells including sperm and in cells of other mammals. In somatic cells, CD46 is present on their surface and it plays a key role in protecting host cells from complement attack - a mechanism which is also used by tumour cells. Another role of CD46 protein is activation of several intracellular pathways in different types of cells including T-lymphocytes. Several pathogens use it as a receptor enabling them to enter the cell. In sperm, CD46 is localized on the head, specifically in an acrosome, and it only becomes surface exposed after a sperm acrosome reaction. The acrosome may be damaged, deformed or disrupted in non-physiological way. If the acrosome is damaged, the sperm is unable to penetrate the egg surroundings and fertilize the egg. The presence of damaged sperm, as well as somatic cells of any kind in the semen is not favourable for successful fertilization.

The role of CD46 in fertilization was already noticed by using monoclonal antibodies to the first short consensus repeat (SCR1) ectodomain of CD46, which resulted in blocking the complement-independent interaction of human sperm with zona-free oocytes/eggs *in vitro.* The recent discovery of a CD46 new human physiological ligand Jagged-1, which is highly expressed by the oocytes/eggs, strengthens the CD46 role in the initial steps of the sperm interaction with oolema (egg plasma membrane). CD46^{-/-}deficient mouse males show a higher rate of spontaneous acrosome reaction compared to wild type males. This finding led to the theory that CD46 plays a role in the stabilization of the acrosomal membrane and consequently the whole acrosome region. CD46 was reported to have a reduced expression in humans with idiopathic infertility.

In humans, the CD46 protein is expressed in all cells except erythrocytes and exists predominantly in four different isoforms - these isoforms are the products of alternative splicing of a single gene. They consist of four extracellular domains on the amino terminus called short consensus repeats (SCRs), a serine-threonine-proline (STP)-rich area, transmembrane domain (TM), intracytoplasmic anchor and one of two forms of cytoplasmic tail (CYT-1 of 16 amino acids and CYT-2 of 23 amino acids) that arise by alternative splicing at carboxyl terminus. The SCR1, 2 and 4 domains are sites of N-linked glycosylation. The STP region is a site of extensive O-linked glycosylation and is composed of 14 or 29 amino acids. The size of the STP region depends on the presence of STP exon B that can be possibly spliced out. Absence of STP exon B leads to lower molecular weight protein isoforms of CD46, while its presence leads to CD46 higher molecular weight isoforms. However, an even lower molecular weight hypoglycosylated isoform of CD46 is expressed in mammalian sperm. In humans, polymorphisms of CD46 gene were detected in normal individuals with both restriction enzymes, HindIII and EcoRI.

### Disclosure of the invention:

The present invention is a novel method for selection and separation of sperm with undamaged intact heads, exploiting the fact that sperm with undamaged intact heads do not present CD46 on their surface in contrast to sperm with damaged heads and the majority of somatic cells, as well as cell debris, which present CD46 on their surface; and on a novel concept of using CD46 as a selector in a simple, fast and effective method.

The present invention provides a method of separation of sperm cells with undamaged intact heads from sperm cells with damaged heads and/or somatic cells and/or cell debris in a cell sample obtained from an animal, comprising the steps of:
- providing an anti-CD46 antibody bound to a carrier and/or to a tag;
- contacting the cell sample with the anti-CD46 antibody bound to the carrier and/or to the tag in order to bind CD46 presenting cells and/or cell debris from the cell sample to the anti-CD46 antibody;
- removing the CD46 presenting cells bound via anti-CD46 antibody to the carrier and/or to the tag to obtain a cell sample free of CD46 presenting cells, wherein CD46 presenting cells include sperm cells with damaged heads, somatic cells, and cell debris.

The present invention is based on utilization of the fact that the CD46 molecule (official full name provided by HGNC - HUGO Gene Nomenclature Committee; http://www.genenames.org/cgi-bin/gene_symbol_report?match=CD46 https://www.ncbi.nlm.nih.gov/gene/4179) is not presented on the surface of undamaged (undisrupted, intact) sperm heads. On the other hand, the sperm with damaged heads (in particular acrosomes) and somatic cells and cell debris present CD46 molecules on their surface. Thus, using anti-CD46 antibody as a selector, unbound sperm with undamaged intact healthy heads can be separated from the sperm selected for damaged heads (namely acrosomes) as well as from somatic cells and debris that are present in a sperm sample, such as an ejaculate or any other sperm containing sample. This allows to separate the undisrupted sperm with undamaged intact heads for further use in assisted reproduction, and to remove the disrupted sperm with damaged heads, somatic cells and debris from a sperm sample.

The present invention thus in a preferred embodiment provides a method of separation of sperm cells with undamaged intact heads from sperm with damaged heads and/or somatic cells and/or cell debris in a sperm sample obtained from an animal, comprising the following steps:
- providing an anti-CD46 antibody bound to a carrier and/or to a tag,
- contacting the sperm sample with the anti-CD46 antibody bound to the carrier and/or to the tag in order to bind CD46 presenting cells and/or cell debris from the sperm sample to the anti-CD46 antibody,
- removing the CD46 presenting cells and cell debris bound via anti-CD46 antibody to the carrier and/or to the tag, thus obtaining the sperm sample with sperm cells with undamaged intact heads.

The animal is preferably a vertebrate such as a mammal or a bird. More preferably, the animal is a non-human animal, a non-human vertebrate, or a non-human mammal.

The term "damaged sperm", "sperm with damaged heads", "sperm with disrupted heads" in this description is intended to designate sperm cells with damaged heads so that CD46 is presented on the surface of the sperm cells and/or is accessible for the anti-CD46 antibody.

The term "sperm with undamaged intact heads" or "undisrupted sperm cells" or "undamaged sperm cells" or "sperm cells with undisrupted heads" or "sperm cells with undamaged heads" designates sperm cells with intact and undisrupted heads, so that CD46 is not presented on the surface of the sperm cells and/or is not accessible for the anti-CD46 antibody.

The term "cell debris" is characterized as cellular components and fragmented remnants of dead or damaged cells or tissues.

The term "cell sample" includes any sample containing a mixture of cells. Preferably, the cell sample is a sperm-containing sample such as an ejaculate, or a pre-treated sperm sample, or a semen sample or a pre-treated semen sample.

The terms "binding" and "bound" include in particular conjugation, covalent binding or non-covalent binding.

The invention enables to separate sperm cells with undamaged intact heads from sperm cells labeled by anti-CD46 antibody for damaged heads (namely damaged acrosome), as well as from somatic cells and cell debris that are present in the sample. The sample may be ejaculate or semen sample (or sperm), or a pre-treated ejaculate or a pre-treated semen sample (or sperm). The pre-treatment may include removal of seminal fluid and/or tissues and/or debris from the ejaculate or from the semen sample or from fresh or conserved sperm, e.g. by density gradient centrifugation or by a swim-up, or by any other suitable method.
The sample containing only sperm cells with undamaged intact heads may further be used in assisted reproduction techniques, including *in vitro* fertilization, intracytoplasmic sperm injection etc., in animal breeding programs and/or in veterinary medicine.

The method of present invention may in some embodiments contain the following steps:
- designing at least one immunization peptide for anti-CD46 antibody production;
- preparing an anti-CD46 antibody;
- binding the anti-CD46 antibody to a carrier and/or to a tag;
- contacting the anti-CD46 antibody bound to the carrier and/or to the tag with a cell sample in order to bind CD46 presenting cells, including sperm cells with damaged heads and/or somatic cells and/or cell debris;
- separating the sperm cells with damaged heads and/or somatic cells and/or cell debris bound to the carrier and/or to the tag via the anti-CD46 antibody from the cell sample;
thereby obtaining two cell fractions: 1) sperm cells with undamaged intact heads and 2) sperm cells with damaged heads and/or somatic cells and/or cell debris.
The method thus results in a selection and separation of sperm cells with damaged heads and/or somatic cells and/or cell debris from the fraction of the sample not presenting CD46.

The "fraction of the sample not presenting CD46" or the "cell sample free of CD46 presenting cells" are used herein as synonyms. This fraction of the sample may include sperm cells with undamaged intact head and/or acellular components of the sample and/or red blood cells. If the initial cell sample is a sperm sample, the fraction of the sample not presenting CD46 contains sperm cells with undamaged intact heads and acellular components of the sample.

Any obtained fraction, i.e., the fraction of the sample not presenting CD46, or the fraction of CD46-presenting cells bound to the carrier and/or to the tag, can be used for further processing as needed, for example in artificial fertilization technologies, animal breeding programs, medical applications, storage purposes, or research.

The CD46 peptides for anti-CD46 antibody production can be the whole CD46 (molecule/protein/peptide/receptor) or its modifications, isoforms and transcript variants (synthetically prepared substances which contain/incorporate a part of the sequence of the molecule) and derivatives (synthetically prepared substances which constitute non-functional subunit of the molecule).

The anti-CD46 antibody may be a commercially available antibody or an antibody produced by known methods. The antibodies may be monoclonal or polyclonal, conjugated or unconjugated, directly or indirectly prepared antibodies against CD46. The anti-CD46 antibodies may be prepared by any suitable method, including, for example, proteomic synthesis, hybridoma cell line technology and immunization by a tissue, cells, whole protein, extracellular part or peptides with/without post-translational modifications. The anti-CD46 antibodies may also be or include synthetic antibodies or derivatives of synthetic antibodies.

For example, the anti-CD46 antibody may be prepared by a method including immunization of animals and/or cells by immunization peptides, which are derived from individual species. In some embodiments, the immunization peptide may be the peptide PFEAMELKGTPKLY (SEQ ID NO. 1).

The carrier may be a substance or device capable of binding the anti-CD46 antibody or its part. The carriers may preferably be in a solid form, in a form of solution or in a form of gel. Examples of suitable carriers include: beads, magnetic beads, polymeric substrates (e.g. polyacrylamide, polystyrene, polycarbonate), cellulosic substrates (e.g., agarose, sepharose), metallic substrates, magnetic or magnetizable carriers. Carriers are typically materials capable of covalent or non-covalent, specifically or non-specifically, directly or indirectly in single or multiple steps binding of the anti-CD46 antibody. The carriers may be applied by coating or immobilization on the surface of materials or devices. In some embodiments, the carriers or the antibodies may be coated or immobilized on the inner surface of a vessel, such as a well, well plate, beaker, capillary or test tube (if antibodies are coated or immobilized on the inner surface of a vessel, then the vessel acts as a carrier).

The contacting step of anti-CD46 antibody with carrier is preferably carried out at about room temperature, in a physiological buffer (such as phosphate buffer, physiological saline solution). The anti-CD46 antibody(ies) bound to the carrier forms an anti-CD46 antibody-carrier complex.

The anti-CD46 antibody-carrier complex allows selection and separation of sperm cells with damaged heads and/or somatic cells and/or cell debris from sperm cells with undamaged intact heads from an ejaculate or from a sperm sample in general. Selection is based on presence or absence of CD46 labeling (meaning absence of CD46 labeling on the surface of sperm with undamaged intact heads).

The tag bound to the anti-CD46 antibody may be selected from fluorophores, chromophores, oligonucleotides, magnetic nanoparticles (nanoparticles having the size from 1 to 1000 nm preferably from 1 to 500 nm, or from 1 to 200 nm), His-tag, MBP-tag, GST-tag, CBP-tag, and Strep-tag. Methods of tagging of antibodies are known in the art.

In case of use of the anti-CD46 antibody-carrier complex, the removal of the CD46 presenting cells is typically based on the removal of the carrier to which the CD46 presenting cells are bound via the anti-CD46 antibody. In case of use of the tagged anti-CD46 antibody (i.e., an anti-CD46 antibody bound to a tag), the removal of the CD46 presenting cells is typically based on cell sorting based on the presence or absence of the tag.

The carrier separation techniques or separation techniques based on the presence or absence of the tag include methods capable to separate carrier comprising anti-CD46 antibody with or without the CD46 presenting cells and methods capable to separate tagged cells from untagged cells. The method may be direct/indirect, passive/active, gradient, biological, biochemical, physical, chemical, enzymatic, non-enzymatic, mechanical. The method may preferably be selected from centrifugation, sedimentation, flow separation methods such as FACS, capillary flow separation, separation on chips, or methods using a magnetic force in case of magnetic or magnetizable carriers and/or tags, for example MACS or magnetic separation. The removal step may also be performed by collecting the fraction of the sample containing the unbound sperm with undamaged intact heads, for example when the carrier is a vessel.

The technology of the present invention is performed *in vitro* or *ex vivo.*

### Brief description of Figures

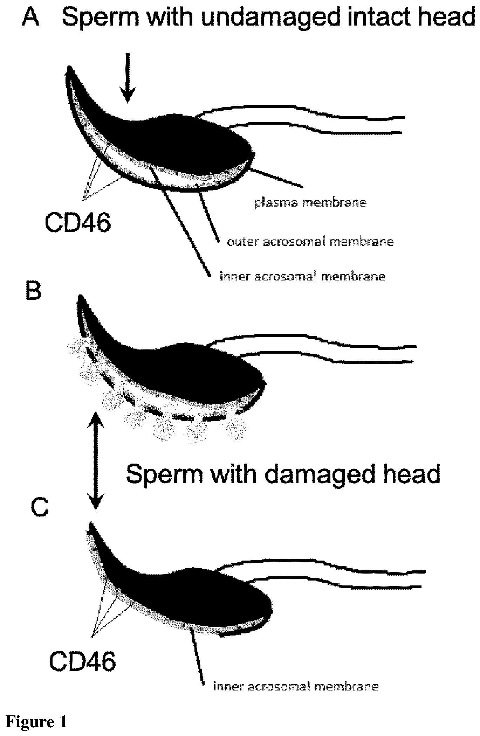

Figure 1: Schematic model of a sperm: (A) sperm with an undamaged intact head and localization of CD46 inside the intact head, which is not accessible for the antibody. (B, C) sperm with the damaged head, wherein CD46 can be detected by a species specific antibody. (B) a partial membrane damage. (C) an excessive head damage with a loss of acrosome.
Figure 2: Mouse epididymal sperm labeled by the anti-CD46 antibody and by Hoechst for the nucleus. Left panel: white signal (arrow) shows damaged sperm labeled by the anti-CD46 antibody; no signal (cross) shows an undamaged sperm with no labeling. Right panel: white signal shows all the sperm in the picture when the labeling for nucleus was used. (The mouse photo documentation is representative for the technology used on all the animals.)
Figure 3: An image of an acrosome of a rat sperm with damaged head, labeled by anti-CD46 antibody.

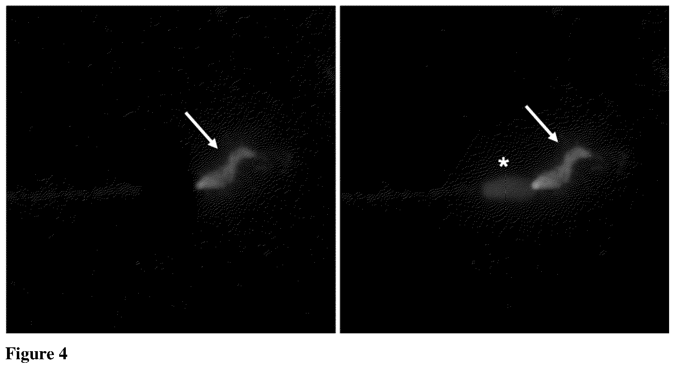

Figure 4: An image of an acrosome of a zebra finch sperm with damaged head, labeled by anti-CD46 antibody. The asterisk marks the nucleus of the sperm.

### Examples of carrying out the invention

### Example 1: Differentiation of sperm with undamaged intact heads from sperm with damaged heads

Mouse epididymal sperm were labeled for nucleus by Hoechst, and for the head integrity by an anti-CD46 primary antibody (clone MM10, HM1118, Hycult Biotech, Uden, Netherland) visualized by a secondary fluorescent antibody (anti-donkey Alexa Fluor 488, Molecular Probes, Prague, Czech Republic). The Hoechst labeling showed all the cells present in the sample, while the labeling by anti-CD46 antibody showed only sperm cells with damaged heads.

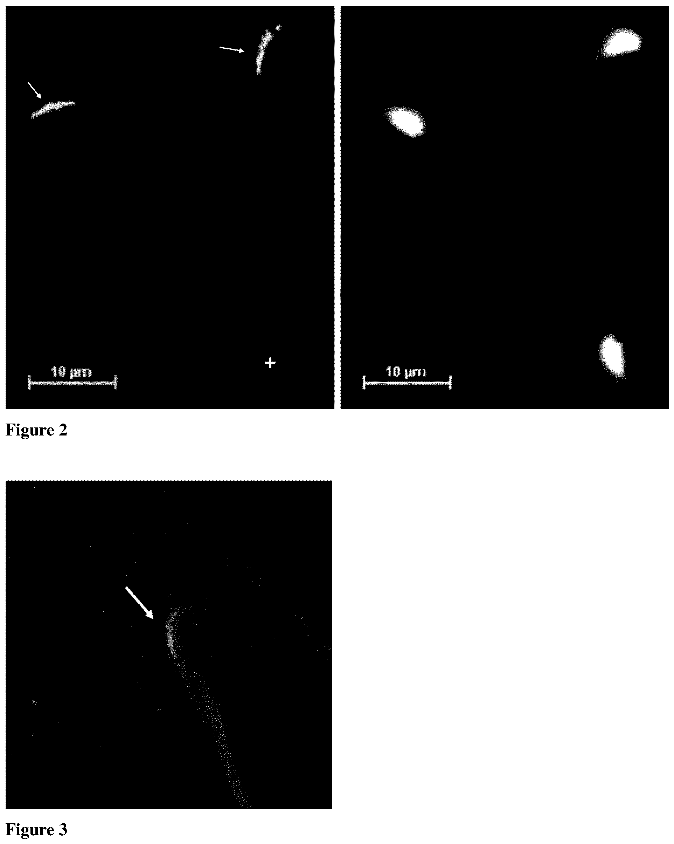

Figure 2 shows an image obtained from the sample - in the left panel, labeling by the anti-CD46 antibody shows the damaged heads of sperm cells. In the right panel, labeling by Hoechst shows all the cells present in the picture. The position of the sperm cell with the undamaged intact head is marked by a cross in the left panel, for better understanding of the images by the reader.

### Protocol 1

A polyclonal anti-CD46 antibody suitable for a number of animal species was prepared as follows:
The polyclonal antibody was prepared on demand by a commercial provider, using immunization of a male New Zealand White (NZW) rabbit. The immunization peptide was PFEAMELKGTPKLY (SEQ ID NO. 1). To prepare the primary injection, 0.75 ml Freunds incomplete adjuvant (FIA) (Sigma-Aldrich, Dorset, UK) was added to 0.75 ml 800 µg/ml peptide-KLH (dissolved in sterile PBS; KLH = keyhole limpet hemocyanin). The mixture was sonicated on ice until a thick stable emulsion developed that did not disperse on the surface of water. The NZW rabbit was injected with a total of 1 ml of the peptide-KLH adjuvant mixture (400 µg) at four subcutaneous sites. Five booster injections were subsequently given over a period of four months. Booster injections comprised of 1ml of 140 µg/ml peptide-KLH adjuvant mixture injected at four subcutaneous sites.

20 ml blood was extracted from the marginal ear vein of the NZW rabbit when collecting pre-immune serum (day 0) and test bleeds (days 39, 46, 53, 68, 81, and 103). 80 ml was extracted during the terminal bleed (day 112). The blood clot was loosened from the side of the universal tube and incubated at 37°C for 1 hour. The universal tube was centrifuged at 7500g for 10 minutes and the serum collected. This step was repeated twice. The serum was incubated at 56°C for 30 minutes to inactivate heat-labile complement components. Serum was stored at - 80°C. Rabbit pre-immune serum (day 0) and rabbit anti-CD46 peptide polyclonal antiserum (day 112) were affinity-purified against the non-conjugated peptide immobilised using a SulfoLink kit (Perbio Science, Cramlington, UK) according to the protocol provided.

Approximately 0.5 mg of free peptide was immobilized to an agarose gel support via a reaction between iodoacetyl groups on the coupling gel and the N-terminal cysteine residue on the peptide. The column was washed and non-specific binding sites on the gel blocked by incubation with L-cysteine. Excess L-cysteine was eluted by washing. The column was incubated with serum and washed to remove non-bound serum proteins. Bound proteins were eluted using 100 mM glycine buffer (pH 2.5-3.0) (Sigma-Aldrich, Dorset, UK) and 1ml fractions were collected. The concentration of the affinity-purified serum was ascertained by absorbance at 280 nm (molar extinction coefficient of 1.4).

### Protocol 2

Material: Cell sample suspension (ejaculate or semen sample), primary species specific antibody anti-CD46 conjugated to a carrier or with a tag.

Method: After a specie-dependent liquefaction of the ejaculate or semen sample, without using any separation method, the ejaculate or semen sample is incubated for 30 minutes with the anti-CD46 antibody conjugated to the carrier or conjugated with the tag, at room temperature, in a physiological buffer (PBS, phosphate buffer saline). Standard procedures and materials were used for binding the antibody to the carrier or to the tag. The incubation of the anti-CD46 antibody-carrier complex or anti-CD46 antibody-tag with the ejaculate or semen sample was followed by separation of the carrier or the tag with the conjugated anti-CD46 antibody to which the CD46 presenting cells are bound. The remaining unbound sperm fraction with undamaged intact heads were transferred to a new container for further use in assisted reproduction (artificial insemination) such as intrauterine insemination (IUI), *in vitro* fertilization (IVF) or intracytoplasmic sperm injection (ICSI), storage purpose, medicine, or any other technique or research.

### Protocol 3

Material: Cryopreserved sample (animal ejaculate or animal semen sample), primary species specific antibody anti-CD46 conjugated to a carrier.

Method: After a sample thawing, the sample is incubated for 30 minutes with the anti-CD46 antibody conjugated to the carrier, at room temperature, in a physiological buffer (PBS, phosphate buffer saline); followed by separation of the carrier with the conjugated anti-CD46 antibody to which the CD46 presenting cells are bound. The remaining unbound sperm fraction with undamaged intact heads were transferred to a new container for further use in assisted reproduction (artificial insemination) such as intrauterine insemination (IUI), *in vitro* fertilization (IVF) or intracytoplasmic sperm injection (ICSI), storage purpose, medicine, or any other technique or research.

### Example 2: Separation of sperm with undamaged intact heads from sperm with damaged heads from the mouse (Mus musculus sp.) semen sample

The anti-mouse CD46 antibody (clone MM10, HM1118, Hycult Biotech, Uden, Netherland) is bound to magnetic beads as a carrier, at room temperature, in a physiological buffer (phosphate buffer) and forms an anti-CD46 antibody-carrier complex. The complex is added to a mouse semen sample, the procedure of Protocol 2 was followed. All the CD46 presenting sperm with damaged heads and all somatic cells, mainly white blood cells, and cell debris are bound to the anti-CD46 antibody-carrier complex and further separated by magnetic field, using a magnetic stand. The unbound sperm with undamaged intact heads remain in the suspension.

The same procedure was repeated with the polyclonal anti-CD46 antibody produced according to Protocol 1. Also with this antibody, all CD46 presenting cells (sperm with damaged heads and all somatic cells, mainly white blood cells, and cell debris) were bound to the carrier via the antibody and removed from the sample by magnetic field. The unbound sperm with undamaged intact heads remained in the suspension.

### Example 3: Separation of sperm with undamaged intact heads from sperm with damaged heads from the rat (Ratus sp.) semen sample

The anti-rat CD46 antibody (clone MM10, HM1118, Hycult Biotech, Uden, Netherland) is bound to agarose beads as a carrier, at room temperature, in a physiological buffer (PBS, phosphate buffer saline). The procedure of Protocol 2 was followed. All the CD46 presenting sperm with damaged heads and all somatic cells, mainly white blood cells, and cell debris are bound to the anti-CD46 antibody-carrier complex and further separated by centrifugation. The unbound sperm with undamaged intact heads remain in the suspension.

The same procedure was repeated with the polyclonal anti-CD46 antibody produced according to Protocol 1. Also with this antibody, all CD46 presenting cells (sperm with damaged heads and all somatic cells, mainly white blood cells, and cell debris) were bound to the carrier via the antibody and removed from the sample by centrifugation. The unbound sperm with undamaged intact heads remained in the suspension.

### Example 4: Separation of sperm with undamaged intact heads from sperm with damaged heads from the pig (Sus sp.) ejaculate

The anti-pig CD46 antibody (clone JM6C11, MCA2262GA, Bio-Rad Laboratories, Hercules, CA) is bound to pre-coated walls of a microcentrifuge tube with a carrier. The procedure of Protocol 2 was followed. All the CD46 presenting sperm with damaged heads and all somatic cells, mainly white blood cells, and cell debris are bound to the anti-CD46 antibody-carrier complex. The unbound sperm with undamaged intact heads remain in the suspension which is transferred into a new container, ready for further use.

### Example 5: Separation of sperm with undamaged intact heads from sperm with damaged heads from the bull (Taurus sp.) ejaculate

The anti-bull CD46 antibody (clone MEM-258, ABIN94152, Antibodies Online, Aachen, Germany) is bound to magnetic beads as a carrier. The procedure of Protocol 2 was followed. All the CD46 presenting sperm with damaged heads and all somatic cells, mainly white blood cells, and cell debris are bound to the anti-CD46 antibody-carrier complex and further separated by magnetic field, using a magnetic stand. The unbound sperm with undamaged intact heads remain in the suspension.

### Example 6: Separation of sperm with undamaged intact heads from sperm with damaged heads from the horse (Equus sp.) ejaculate

The anti-horse CD46 antibody (ab231984, Abcam, Cambridge, UK) is bound to polyacrylamide beads. The procedure of Protocol 2 was followed. All the CD46 presenting sperm with damaged heads and all somatic cells, mainly white blood cells, and cell debris are bound to the anti-CD46 antibody-carrier complex and further separated by centrifugation. The unbound sperm with undamaged intact heads remain in the suspension.

### Example 7: Separation of sperm with undamaged intact heads from sperm with damaged heads from the canine (Canis sp.) ejaculate

The anti-dog CD46 antibody (E4.3, Santa Cruz Biotechnology, Inc., Santa Cruz, CA) is bound to magnetic beads as a carrier. The procedure of Protocol 2 was followed. All the CD46 presenting sperm with damaged heads and all somatic cells, mainly white blood cells, and cell debris are bound to the anti-CD46 antibody-carrier complex and further separated by magnetic field. The unbound sperm with undamaged intact heads remain in the suspension.

### Example 8: Separation of sperm with undamaged and intact heads from sperm with damaged heads in sperm sample from the cryopreserved pig (Sus sp.) semen sample

The procedure of Protocol 3 was followed, using the antibody and carrier as specified in Example 4. All the CD46 presenting sperm with damaged heads and all somatic cells, mainly white blood cells, and cell debris are bound to the anti-CD46 antibody-carrier complex. The unbound sperm with undamaged intact heads remain in the suspension which is transferred into another container, and they are ready to be used for short or long-term storage under any conditions and media such as cryopreservation or any kind of sample preservation.

### Example 9: Separation of sperm with undamaged and intact heads from sperm with damaged heads from the cryopreserved bull (Taurus sp.) semen sample

The procedure of Protocol 3 was followed, using the antibody and carrier as specified in Example 5. All the CD46 presenting sperm with damaged heads and all somatic cells, mainly white blood cells, and cell debris are bound to the anti-CD46 antibody-carrier complex and further separated by magnetic field. The unbound sperm with undamaged intact heads remain in the suspension and they are ready to be used for short or long-term storage under any conditions and media such as cryopreservation or any kind of sample preservation.

### Example 11: Separation of sperm with undamaged and intact heads from sperm with damaged heads from the sperm sample of zebra finch (Taeniopygia guttata)

The polyclonal antibody produced according to Protocol 1 was bound to magnetic beads and was used in the procedure of Protocol 2. All the CD46 presenting sperm with damaged heads and all somatic cells, mainly white blood cells, and cell debris are bound to the anti-CD46 antibody-carrier complex and separated by magnetic field, using a magnetic stand.

### Industrial Applicability

The present invention is a technology for selection and separation of CD46 presenting components from cells not presenting CD46 in cell samples obtained from an animal. The cells not presenting CD46 are sperm with undamaged intact heads. The technology may thus serve for separation of sperm with undamaged intact heads from sperm with damaged heads, as well as from somatic cells and cell debris in a sperm sample. This technology is designed to separate sperm with undamaged intact heads to be further used in assisted reproduction, medicine (in particular breeding programs and veterinary medicine) and research. The invention results in an increased quality of the sperm cell sample, as well as allows separation of somatic cells and/or debris from sperm-containing samples. Such purified samples result in an increased fertilization or medical or research success. The invention also results in an increased quality of the sperm sample for long- or short-term storage under any condition and media such as cryopreservation. This invention also saves large amount of cryoprotectives and plastic by storage of the pre-selected quality sample.

## Claims

1. A method of separation of sperm cells with undamaged intact heads from sperm cells with damaged heads and/or somatic cells and/or cell debris in a cell sample obtained from an animal, comprising the steps of:
- providing an anti-CD46 antibody bound to a carrier and/or to a tag;
- contacting the cell sample with the anti-CD46 antibody bound to the carrier and/or to the tag in order to bind CD46 presenting cells and/or cell debris from the cell sample to the anti-CD46 antibody;
- removing the CD46 presenting cells bound via the anti-CD46 antibody to the carrier and/or to the tag to obtain a cell sample free of CD46 presenting cells, wherein CD46 presenting cells include sperm cells with damaged heads, somatic cells and cell debris.

2. The method according to claim 1, wherein the starting cell sample is a sperm sample, and wherein the cell sample free of CD46 presenting cells is a sperm containing only sperm cells with undamaged intact heads.

3. The method according to claim 1 or 2, wherein the starting cell sample is an ejaculate or a pre-treated ejaculate or a semen sample or a pre-treated semen sample, wherein the pre-treatment preferably includes removal of seminal fluid and/or tissues and/or cell debris from the ejaculate.

4. The method according to any one of the preceding claims, which contains the following steps:
- providing at least one immunization peptide for anti-CD46 antibodies production;
- producing an anti-CD46 antibody;
- binding the anti-CD46 antibody to a carrier and/or to a tag;
- contacting the cell sample with the anti-CD46 antibody bound to the carrier and/or to the tag with a cell sample in order to bind CD46 presenting cells, including sperm cells with damaged heads and/or somatic cells and/or cell debris, from the cell sample to the anti-CD46 antibody;
- separating the sperm cells with damaged heads and/or somatic cells and/or cell debris, bound via the anti-CD46 antibody to the carrier and/or to the tag, from the cell sample;
thereby obtaining two cell fractions: 1) sperm cells with undamaged intact heads and 2) sperm cells with damaged heads and/or somatic cells and/or cell debris.

5. The method according to claim 4, wherein the anti-CD46 antibody is produced by immunization of a production animal by an immunization peptide having the sequence PFEAMELKGTPKLY (SEQ ID NO. 1).

6. The method according to any one of the preceding claims, wherein the carrier is selected from the group comprising beads, magnetic beads, polymeric substrates, cellulosic substrates, metallic substrates, magnetic or magnetizable carriers.

7. The method according to any one of claims 1-5, wherein the carrier is a vessel, such as a well, well plate, beaker, capillary or test tube, wherein the anti-CD46 antibody is coated or immobilized on the inner surface of the vessel; or wherein the carrier is coated or immobilized on the inner surface of a vessel, such as a well, well plate, beaker, capillary or test tube.

8. The method according to any one of the preceding claims, wherein the removal of the CD46 presenting cells bound via anti-CD46 antibody to the carrier and/or to the tag is carried out by centrifugation, sedimentation, flow separation methods such as FACS, capillary flow separation, separation on chips, or by use of magnetic force such as MACS or magnetic separation.

9. Use of anti-CD46 antibody for *in vitro* separation of sperm cells with undamaged intact heads from sperm cells with damaged heads and/or somatic cells and/or cell debris in a cell sample, preferably a sperm or semen sample, obtained from an animal.


SEQUENCE LISTING

<110>  Biotechnologicky ustav AV CR, v. v. i.; Ceska zemedelska
       univerzita v Praze; PrimeCell Advanced Therapy a.s.

<120>  Method for separation of sperm with undamaged intact heads from
       sperm with damaged heads and somatic cells

<130>  P

<160>  1

<170>  PatentIn version 3.5

<210>  1
<211>  14
<212>  PRT
<213>  artificial

<220>
<223>  Artificially assembled immunization peptide

<400>  1

Pro Phe Glu Ala Met Glu Leu Lys Gly Thr Pro Lys Leu Tyr
1               5                   10